# EUROPEAN PATENT SPECIFICATION

(11) **EP 3 138 526 B1**
(45) Date of publication and mention of the grant of the patent: **19.09.2018**
(21) Application number: 15172493.7
(22) Date of filing: 17.06.2015
(51) Int. Cl.: A61B 34/10, A61B 90/00

(54) **AUGMENTED SURGICAL REALITY ENVIRONMENT SYSTEM**
CHIRURGISCHES SYSTEM MIT ERWEITERTER REALITÄT
SYSTÈME D'ENVIRONNEMENT DE RÉALITÉ CHIRURGICALE AUGMENTÉE

(30) Priority: 18.06.2014 US 201462013604 P; 12.05.2015 US 201514709800
(43) Date of publication of application: 08.03.2017
(73) Proprietor: Covidien LP, Mansfield, MA 02048 (US)
(72) Inventor: INGMANSON, Michael, Stratford, CT Connecticut 06614 (US)
(74) Representative: Soames, Candida Jane

(56) References cited:
- WO-A2-2010/102197
- JP-B2- 3 024 162
- US-A1- 2002 077 533
- US-A1- 2006 176 242
- US-A1- 2012 059 249

## Description

### BACKGROUND

### 1. Technical Field

The present disclosure relates to minimally invasive surgical techniques to improve patient outcome. More specifically, the present disclosure is directed to systems and methods for augmenting and enhancing a clinician's field of vision while performing a minimally invasive surgical technique.

### 2. Background of the Related Art

Today, many surgical procedures are performed through small openings in the skin, as compared to the larger openings typically required in traditional procedures, in an effort to reduce both trauma to the patient and recovery time. Such procedures are known as "minimally invasive" procedures. During the course of minimally invasive procedures, the nature of the relatively small opening through which surgical instruments are manipulated, and/or the presence of sub-surface tissue structures, may obscure a direct line-of-sight to the target surgical site. As such, a clinicians' field of vision, intuitive orientation, and spatial comprehension are limited. Therefore, there is a need to improve the field of vision as well as incorporate advanced and supplemental information to aid the clinician.

US 2012/0059249 A1 discloses an image guided navigation system for navigating a region of a subject, the system including an imaging device, a tracking device, a controller, and a display.

WO 2010/102197 A2 discloses an apparatus for monitoring a thermal surgical procedure, the apparatus including a thermal camera, a processor, and a display.

### SUMMARY

The present invention is disclosed in claim 1. In an embodiment of the present disclosure, an augmented surgical reality environment system is provided. The system includes an image capture device configured to capture an image of a surgical environment and at least one biometric sensor configured to obtain biometric data from a patient. The system also includes a controller having a memory configured to store a plurality of anatomical images and a processor. The processor receives at least one of the captured image, the biometric data, or one or more anatomical images from the plurality of anatomical images and generates an augmented image from at least one of the captured image, the biometric data, or the one or more anatomical images. A display device displays the augmented image.

In some aspects, the display device is a projector, a laser based system, or a monitor. In other aspects, the display device includes a frame having at least one lens and a projector configured to project the augmented image onto the lens.

In aspects, the image capture device is a camera.

In some aspect described herein, the augmented image includes organs or body structures.

In other aspects, the controller determines a position or orientation of a surgical tool relative to the patient and the augmented image includes a virtual image of a portion of the surgical tool disposed within the patient. The position or orientation of the surgical tool is determined based on an image of the surgical tool captured by the image capture device or the position or orientation of the surgical tool is determined by data provided by the surgical tool. The data provided by the surgical tool includes accelerometer data or gyroscopic data.

In other aspects, the image capture device captures an image of an object in the surgical environment. The processor determines a position of the object relative to the patient based on the image of the object. The augmented image includes an enhanced representation of the object and the display device displays the enhanced representation on the patient at the position determined by the processor. In other aspects, the controller receives a position signal from an object and the processor determines a position of the object based on the received position signal. The augmented image includes an enhanced representation of the object and the display device displays the enhanced representation on the patient at the position determined by the processor.

In aspects, the plurality of anatomical images are obtained from an x-ray, a computed tomography scan, or magnetic resonance imaging data. The anatomical images are processed by the processor to enhance a portion of the anatomical image. The enhanced portion of the anatomical image is displayed on the patient by the display device. The enhanced portion of the anatomical image may include a heat map.

In some aspects, the biometric data includes one or more vital signs of the patient. A virtual representation of the one or more vital signs is included in the augmented image. A color of the virtual representation is changed based on a value of the one or more vital signs.

In some aspects, the augmented image includes a surgical plan which includes at least one of a cut path, incision location, implant location, or notes.

In other aspects, the system includes a surgical device and the augmented image includes a status of the surgical device.

In some aspects, the captured image includes a direction and magnitude of a first cut and the processor determines a desired cut path and a distance for a second cut based on the direction and magnitude of the first cut and the plurality of anatomical images stored in the memory. The augmented image includes an image representing a direction and magnitude of the second cut.

In other aspects, the image capture device captures a first image and a second image. The controller determines if an object has moved based on a difference between the first image and the second image. The controller highlights the object in the augmented image to be displayed on the display.

In other aspects the memory stores a plurality of tools to be used and an order of use for the plurality tools during a surgical procedure. The controller determines a tool among the plurality of tools has been used based on the image from the image capture device. The controller determines a tool among the plurality of tools to be used based on the order of use for the plurality of tools and the tool that has been used. The controller highlights the tool to be used in the augmented image.

In another embodiment of the present disclosure, which is not part of the invention, a method for augmenting an image of a surgical environment is provided. The method involves obtaining anatomical image data from a memory and displaying the anatomical image over a patient. A region of interest in the anatomical image is selected, highlighted, and displayed.

In some aspects, the anatomical image may be manipulated and displayed.

In yet another embodiment of the present disclosure, which is not part of the invention, another method for augmenting an image of a surgical environment is provided. The method involves capturing image data and identifying a surgical device and a first location of the surgical device with respect to a patient in the image data. An augmented image including the surgical device at the first location is displayed over the patient.

In some aspects, the surgical device is moved and a second location of the surgical device with respect to the patient is calculated. The surgical device is displayed at the second location over the patient.

In yet another embodiment of the present disclosure, which is not part of the invention, a method for augmenting an image of a surgical environment is provided that involves capturing image data and identifying an object and a first location of the object with respect to a patient in the image data. An augmented image including an indicator representative of the object is displayed at the first location over the patient.

In some aspects, when the object has moved, a second location of the object calculated with respect to the patient and the indicator is displayed at the second location over the patient. When the object has not been removed from the patient, the display continues to display the indicator over the patient until the object is removed from the patient.

In yet another embodiment, which is not part of the invention, a method for augmenting an image of a surgical environment is provided. The method involves obtaining biometric data from a patient and determining when the biometric data is within a predetermined range. An augmented image including the biometric data is displayed, wherein the biometric data is displayed in a first color when the biometric data is within the predetermined range, and the biometric data is displayed in a second color when the biometric data is outside the predetermined range.

The biometric data is at least one of pulse, temperature, blood pressure, blood oxygen levels, or heart rhythm.

In yet another embodiment, which is not part of the invention, a method for augmenting an image of a surgical environment is provided. The method involves obtaining device status from a surgical device and determining when the device status is within a predetermined range. An augmented image including the device status displayed, wherein the device status is displayed in a first color when the device status is within the predetermined range, and the device status is displayed in a second color when the device status is outside the predetermined range.

The device status is at least one of firing range, remaining device life, battery charge, tissue thickness, or tissue impedance.

Further details and aspects of exemplary embodiments of the present disclosure are described in more detail below with reference to the appended figures.

### BRIEF DESCRIPTION OF THE DRAWINGS

The above and other aspects, features, and advantages of the present disclosure will become more apparent in light of the following detailed description when taken in conjunction with the accompanying drawings in which:
Fig. 1 is a system block diagram of a system for augmenting a surgical environment in accordance with an embodiment of the present disclosure;
Figs. 2A-2D are examples of how the system of Fig. 1 may be implemented in accordance with embodiments of the present disclosure;
Fig. 3 depicts an augmented image in accordance with an embodiment of the present disclosure;
Fig. 4 is a flow chart depicting the process for obtaining the augmented image of Fig. 3;
Fig. 5 depicts an augmented image in accordance with another embodiment of the present disclosure;
Fig. 6 is a flow chart depicting the process for obtaining the augmented image of Fig. 5;
Fig. 7 depicts an augmented image in accordance with another embodiment of the present disclosure;
Fig. 8 is a flow chart depicting the process for obtaining the augmented image of Fig. 7;
Fig. 9 depicts an augmented image that is overlaid on a laparoscopic video in accordance with another embodiment of the present disclosure;
Fig. 10 depicts an augmented image that is overlaid on a patient in accordance with another embodiment of the present disclosure;
Fig. 11 depicts an augmented image in accordance with another embodiment of the present disclosure;
Fig. 12 is a flow chart depicting the process for obtaining the augmented image of Fig. 11;
Fig. 13 depicts an augmented image in accordance with another embodiment of the present disclosure; and
Fig. 14 is a flow chart depicting the process for obtaining the augmented image of Fig. 13.

### DETAILED DESCRIPTION

Particular embodiments of the present disclosure are described hereinbelow with reference to the accompanying drawings; however, it is to be understood that the disclosed embodiments are merely examples of the disclosure and may be embodied in various forms. Well-known functions or constructions are not described in detail to avoid obscuring the present disclosure with unnecessary detail. Therefore, specific structural and functional details disclosed herein are not to be interpreted as limiting, but merely as a basis for the claims and as a representative basis for teaching one skilled in the art to variously employ the present disclosure in virtually any appropriately detailed structure. Like reference numerals refer to similar or identical elements throughout the description of the figures.

This description may use the phrases "in an embodiment," "in embodiments," "in some embodiments," or "in other embodiments," which may each refer to one or more of the same or different embodiments in accordance with the present disclosure. For the purposes of this description, a phrase in the form "A or B" means "(A), (B), or (A and B)". For the purposes of this description, a phrase in the form "at least one of A, B, or C" means "(A), (B), (C), (A and B), (A and C), (B and C), or (A, B and C)".

The term "clinician" refers to any medical professional (i.e., doctor, surgeon, nurse, or the like) performing a medical procedure involving the use of embodiments described herein. As shown in the drawings and described throughout the following description, as is traditional when referring to relative positioning on a surgical instrument, the term "proximal" or "trailing" refers to the end of the apparatus which is closer to the clinician and the term "distal" or "leading" refers to the end of the apparatus which is further away from the clinician.

The systems described herein may also utilize one or more controllers to receive various information and transform the received information to generate an output. The controller may include any type of computing device, computational circuit, or any type of processor or processing circuit capable of executing a series of instructions that are stored in a memory. The controller may include multiple processors and/or multicore central processing units (CPUs) and may include any type of processor, such as a microprocessor, digital signal processor, microcontroller, or the like. The controller may also include a memory to store data and/or algorithms to perform a series of instructions.

Any of the herein described methods, programs, algorithms or codes may be converted to, or expressed in, a programming language or computer program. A "Programming Language" and "Computer Program" is any language used to specify instructions to a computer, and includes (but is not limited to) these languages and their derivatives: Assembler, Basic, Batch files, BCPL, C, C+, C++, Delphi, Fortran, Java, JavaScript, Machine code, operating system command languages, Pascal, Perl, PL1, scripting languages, Visual Basic, metalanguages which themselves specify programs, and all first, second, third, fourth, and fifth generation computer languages. Also included are database and other data schemas, and any other metalanguages. For the purposes of this definition, no distinction is made between languages which are interpreted, compiled, or use both compiled and interpreted approaches. For the purposes of this definition, no distinction is made between compiled and source versions of a program. Thus, reference to a program, where the programming language could exist in more than one state (such as source, compiled, object, or linked) is a reference to any and all such states. The definition also encompasses the actual instructions and the intent of those instructions.

Any of the herein described methods, programs, algorithms or codes may be contained on one or more machine-readable media or memory. The term "memory" may include a mechanism that provides (e.g., stores and/or transmits) information in a form readable by a machine such a processor, computer, or a digital processing device. For example, a memory may include a read only memory (ROM), random access memory (RAM), magnetic disk storage media, optical storage media, flash memory devices, or any other volatile or non-volatile memory storage device. Code or instructions contained thereon can be represented by carrier wave signals, infrared signals, digital signals, and by other like signals.

The present disclosure is directed to systems and methods for providing an augmented surgical reality environment to a clinician during a minimally invasive surgical procedure. The systems and method described herein utilize captured image data, anatomical image data, and/or biometric data to provide an augmented or enhanced image to a clinician via a display. Providing the augmented image to the clinician results in improved dexterity, improved spatial comprehension, potential for more efficient removal of tissue while leaving healthy tissue intact, improved port placement, improved tracking, reducing loss of objects in a patient, and reducing duration of a surgical procedure.

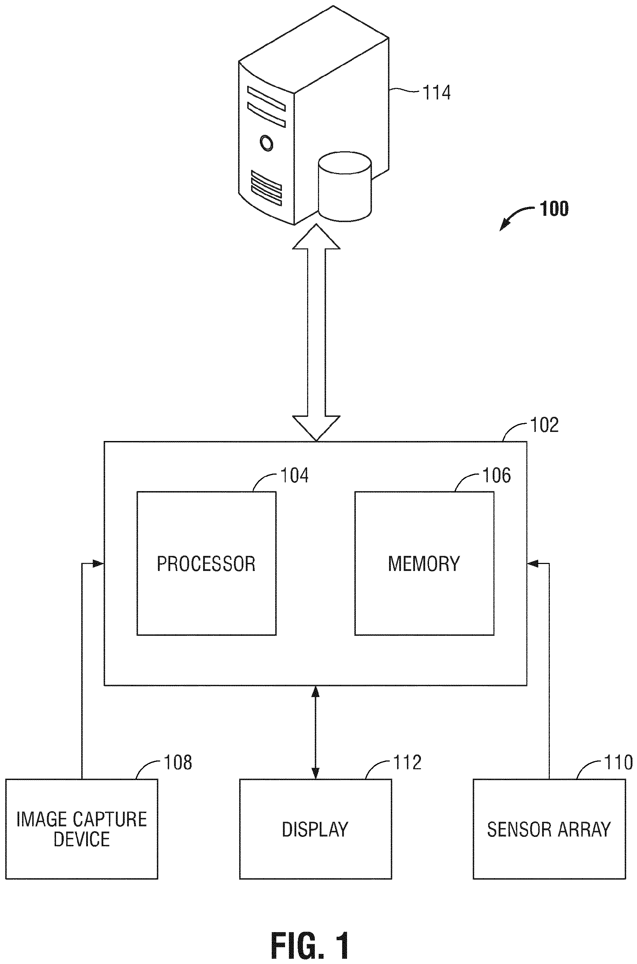

Turning to Fig. 1, a system for augmenting a surgical environment, according to embodiments of the present disclosure, is shown generally as 100. System 100 includes a controller 102 that has a processor 104 and a memory 106. The system 100 also includes an image capture device 108, e.g., a camera, that records still frame images or moving images. A sensor array 110 provides information concerning the surgical environment to the controller 102. For instance, sensor array 110 includes biometric sensors capable of obtaining biometric data of a patient such as, pulse, temperature, blood pressure, blood oxygen levels, heart rhythm, etc. A display 112, displays augmented images to a clinician during a surgical procedure. The controller 102 communicates with a central server 114 via a wireless or wired connection. Alternatively, controller 102 may communicate with central server 114 before a surgical procedure. The server 114 stores images of a patient or multiple patients that may be obtained using x-ray, a computed tomography scan, or magnetic resonance imaging.

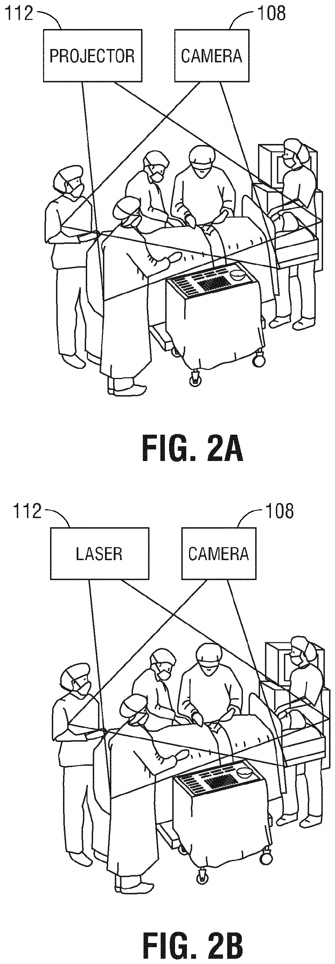

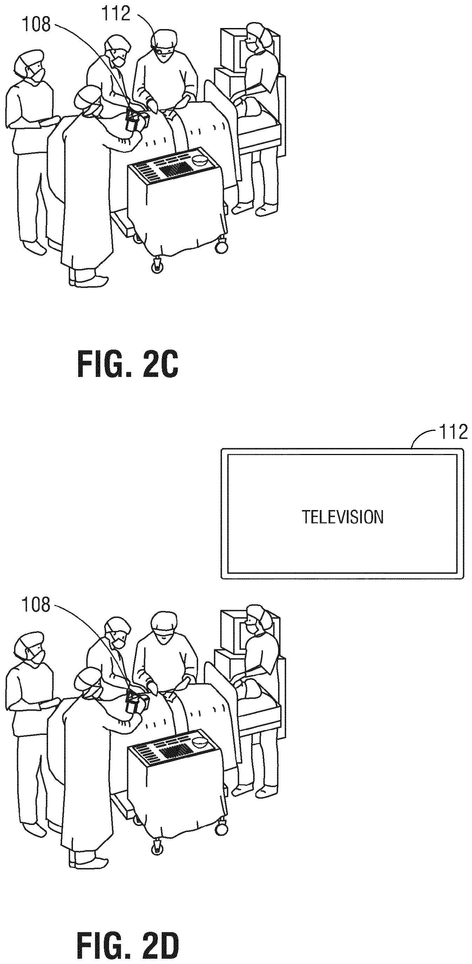

Figs. 2A-2D depict examples of how the system of Fig. 1 is implemented in a surgical environment. As shown in Figs. 2A-2D, an image capture device 108 captures images of a surgical environment during a surgical procedure. Images recorded by the image capture device 108, data from the sensor array 110, and images from server 114 are combined by the controller 102 to generate an augmented image that is provided to a clinician via display 112. As shown in Figs. 2A-2D, display 112 may be a projector (Fig. 2A), a laser projection system (Fig. 2B), a pair of glasses that projects an image onto one of the lenses such as GOOGLE GLASS® (provided by Google®) (Fig. 2C), or a monitor (Fig. 2D). When using a monitor as shown in Fig. 2D, the augmented image is overlaid on an image of the patient obtained by the image capture device 108.

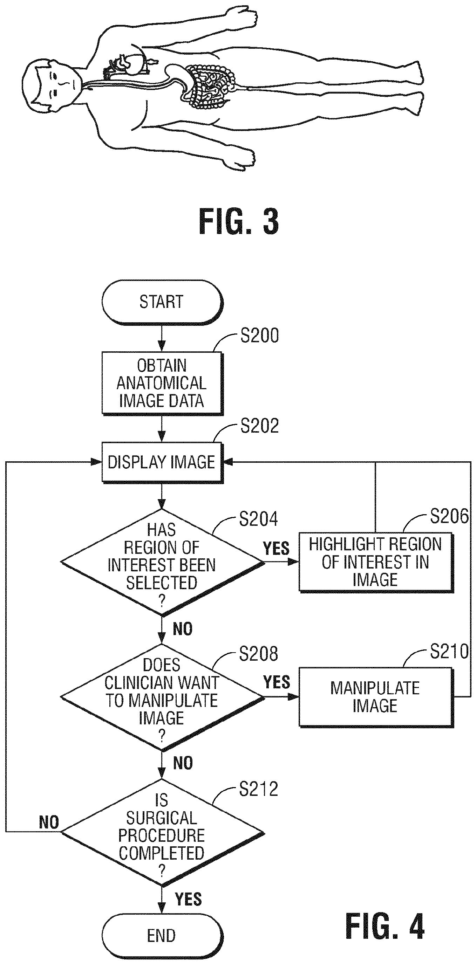

System 100 of Fig. 1 can be used to overlay anatomical images of a patient during a surgical procedure as shown in Fig. 3. Because minimally invasive surgery uses a small incision and ports to gain access to internal body structures, the clinicians field of view is often hampered. The system of Fig. 1 can be used to show the locations of internal body structures to increase a clinician's field of view and provide optimal port placement.

Fig. 4 depicts a schematic process for overlaying images of a patient on the patient. In step s200, anatomical image data is obtained from memory 106 or server 114. In many instances, memory 106 may obtain the anatomical images from server 114 in advance of the surgical procedure. The controller 102 receives image data of the patient from image capture device 108 and aligns that anatomical image to the location of the patient. The alignment may be performed based on matching external body structures of the patient to the anatomical images or the use of fiducial markers that are placed in the anatomical images and on the patient. The display 112 then displays the anatomical image on the patient in step s202. In step s204, the controller 102 determines if the clinician wants to highlight a region of interest. Image capture device 108 captures hand gestures of a clinician and the controller 102 determines a region of interest selected by the clinician based on the hand gestures. If the clinician selects a region of interest, the process proceeds to step s206 where the region of interest is highlighted and then the image is displayed again in step s202. If the clinician does not select a region of interest, the process proceeds to step s208 where the controller 102 determines whether the clinician wants to manipulate the image. Manipulating the image may involve zooming in/out of a particular area or manipulating the orientation of the image in 2-dimensional or 3-dimensional space. With regard to zooming in/out, the clinician may make simple hand gestures that are captured by the image capture device 108 and interpreted by the controller 102 to zoom in/out. With regard to manipulating the orientation of the image, the clinician may use simple hand gestures or the clinician may adjust the patient at different orientations. The image capture device 108 would capture an image of the hand gestures or patient in the new orientation and provide the image to the controller 102. In step s210, the controller 102 manipulates the image according to the hand gestures or patient orientation and provides the manipulated image to the display 112. Then the process returns to step s202 where the image is displayed. If the controller 102 determines that image does not need manipulation, the process proceeds to step s212 where the controller determines if the surgical procedure is completed. If the surgical procedure is not completed, the process returns to step s202 and continues to display the anatomical image.. If the procedure is completed the process ends.

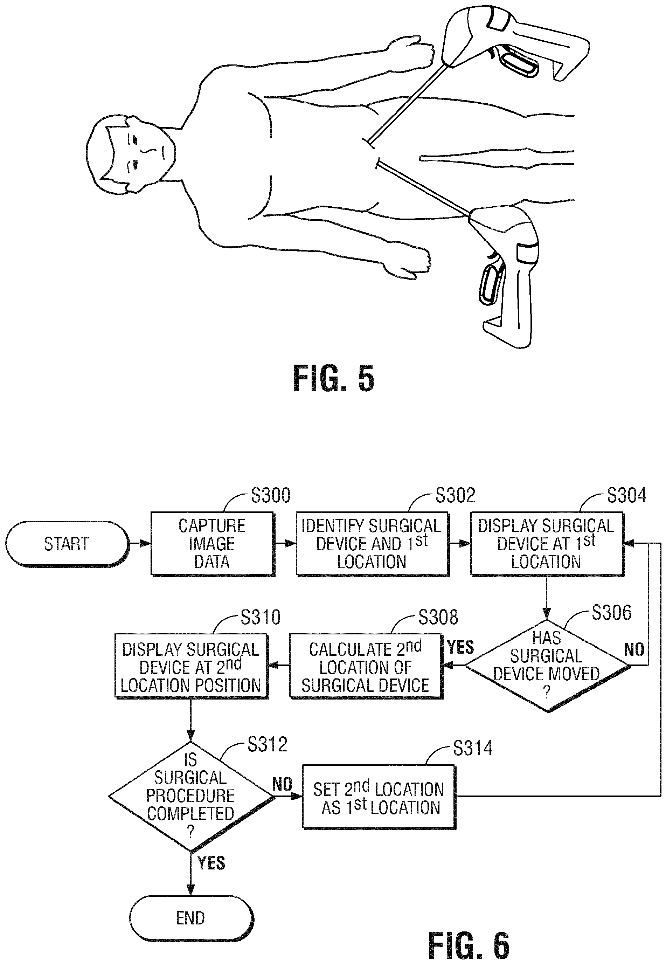

System 100 of Fig. 1 can also be used to display a portion of a surgical device that is obscured from a clinician's field of view because it is inserted into a patient as shown in Fig. 5. Fig. 6 is a flowchart depicting the process for displaying the surgical device on a patient. The process beings in step s300 where the image capture device 108 captures an image of the surgical environment. The controller 102 then identifies one or more surgical devices within the surgical environment in step s302. Identification of the surgical device includes matching a profile of the device to a profile stored in memory 106. In some embodiments, the device may include a 2-dimensional or 3-dimensional bar code that is recognized by the controller 102. The controller 102 also determines a 1^{st} location of the surgical device in relation to the patient. Controller 102 then transmits an image of the surgical device to be displayed on display 112. The image of the surgical device is aligned with the surgical device based on the 1^{st} location and the portion of the surgical device that is inserted into the patient is displayed on the exterior surface of the patient in step s304. In step s306, the controller 102 determines if the surgical device has moved based on images from the image capture device 108. If the surgical device has not moved, the process returns to step s304 and display of the surgical device at the 1^{st} location is continued. If the surgical device has moved, the process proceeds to step s308 where a 2^{nd} location of the surgical device is calculated in relation to the patient. In step s310, the surgical device is displayed on the patient at the 2^{nd} location. If the controller 102 determines that the surgical procedure is not completed in step s312, the process proceeds to step s314 where the 2^{nd} location of the surgical device is stored as the first location. Then the process returns to step s304 to display the surgical device at the first location. Otherwise, if the surgical procedure is completed, the process ends.

In some embodiments, a position and orientation of the surgical device is provided by accelerometer data or gyroscopic data provided by the surgical device instead of the image captured by the image capture device 108.

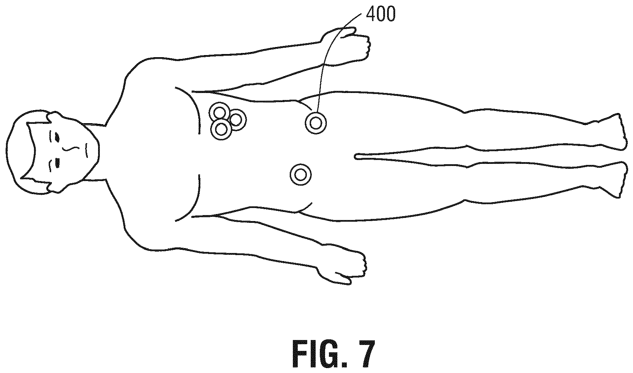

In the past, there have been many instances of clinicians leaving foreign bodies or objects, e.g., sponges, gauze, tools, etc., in a patient after the procedure has ended and all openings have been sealed. This has led to complications in the patient's recovery. Thus, the embodiment of Fig. 1 can be used to reduce or eliminate the number of foreign bodies or objects left behind in a patient. Particularly, the system 100 can track the objects and provide an augmented image which includes the location of all objects in the surgical environment as shown in Fig. 7. The objects can be displayed using an enlarged visible indicator 400.

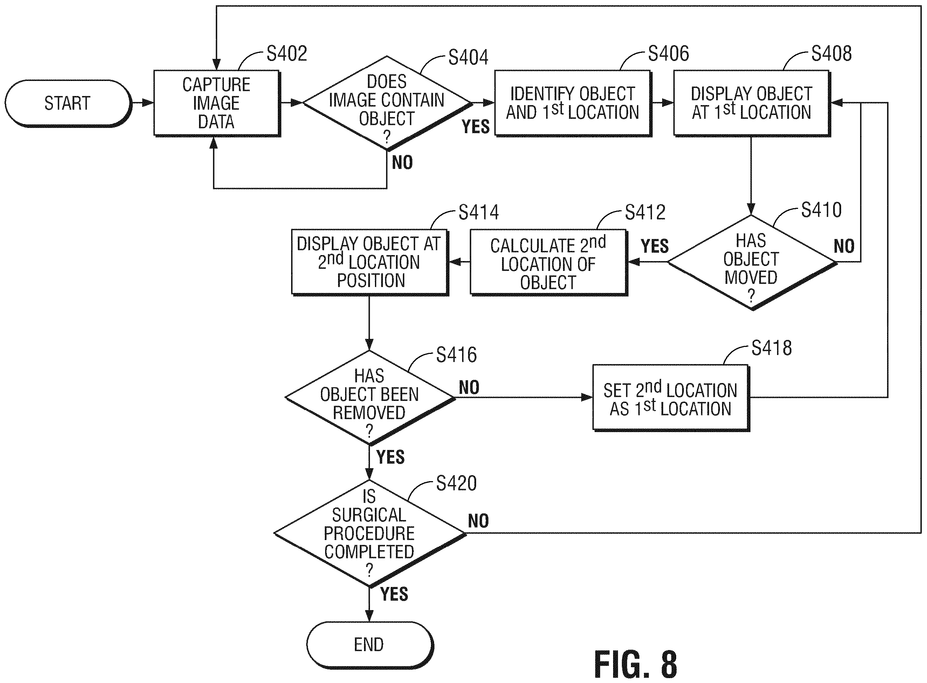

Fig. 8 is a flowchart depicting the method for acquiring and tracking an object or multiple objects. The process beginning in step s402 where the image capture device 108 captures an image of the surgical environment. The controller 102 determines whether there is an object in the captured image in step s404. If there is no object in the image, the process returns to step s402. If there is an object in the image, the controller 102 then identifies the object(s) within the surgical environment in step s406. Identification of the object includes matching a profile of the device to a profile stored in memory 106. In some embodiments, the device may include a 2-dimensional or 3-dimensional bar code that is recognized by the controller 102. The controller 102 also determines a 1^{st} location of the object in relation to the patient. Controller 102 then transmits an image of the object to be displayed on display 112. The image of the object is aligned with the patient based on the 1^{st} location and displayed in step s408. In step s410, the controller 102 determines if the object has moved based on images from the image capture device 108. If the object has not moved, the process returns to step s408 and display of the object at the 1^{st} location is continued. If the object has moved, the process proceeds to step s412 where a 2^{nd} location of the object is calculated in relation to the patient. In step s414, the object is displayed on the patient at the 2^{nd} location. If the controller 102 determines that the object has not been removed in step s416, the process proceeds to step s418 where the 2^{nd} location is set as the 1^{st} location and the object is displayed at the first location in step s408. If the object has been removed, the process proceeds to step s420 where the controller 102 determines whether the surgical procedure is completed. If the procedure is not completed, then the process returns to step s402. Otherwise, if the surgical procedure is completed, the process ends.

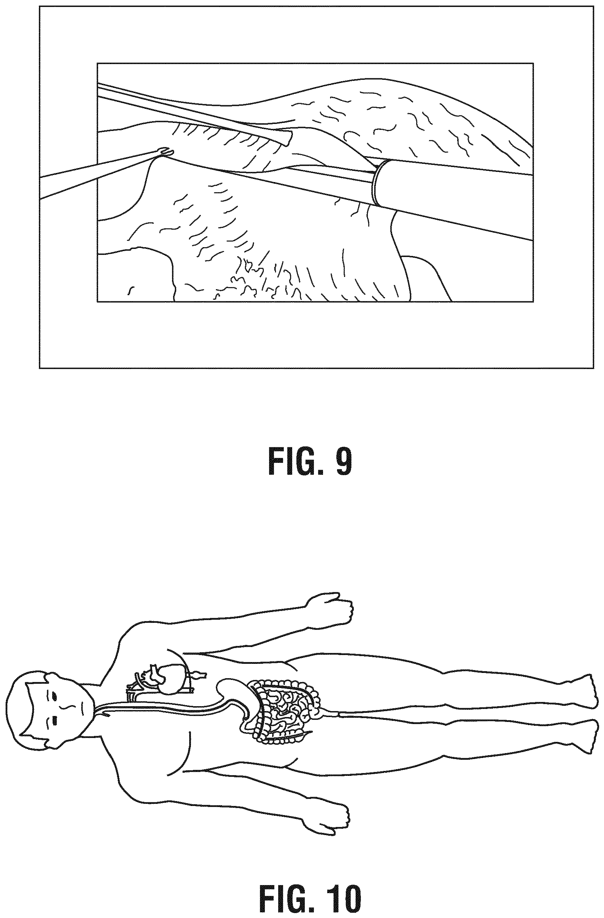

System 100 may also be used to overlay diagnostic data onto a patient as shown in Figs. 9 and 10. Fig. 9 depicts data that is overlaid using laparoscopic video while Fig. 10 depicts data that is displayed externally. In some embodiments, past diagnostic results may be overlaid onto the patient. In other embodiments, X-ray, CT scan, and MRI images may be interpreted before a surgical procedure. The interpreted images are stored in server 114 and transferred to memory 106 before the surgical procedure. The interpreted images may be color coded to make a heat map, e.g., a heat map of cancerous tissue. The clinician may then view the image in real time permitting the clinician to identify the regions to which any cancer has spread thereby increasing the efficacy of cancerous tissue removal. This results in an increase in the amount of good tissue that may be saved.

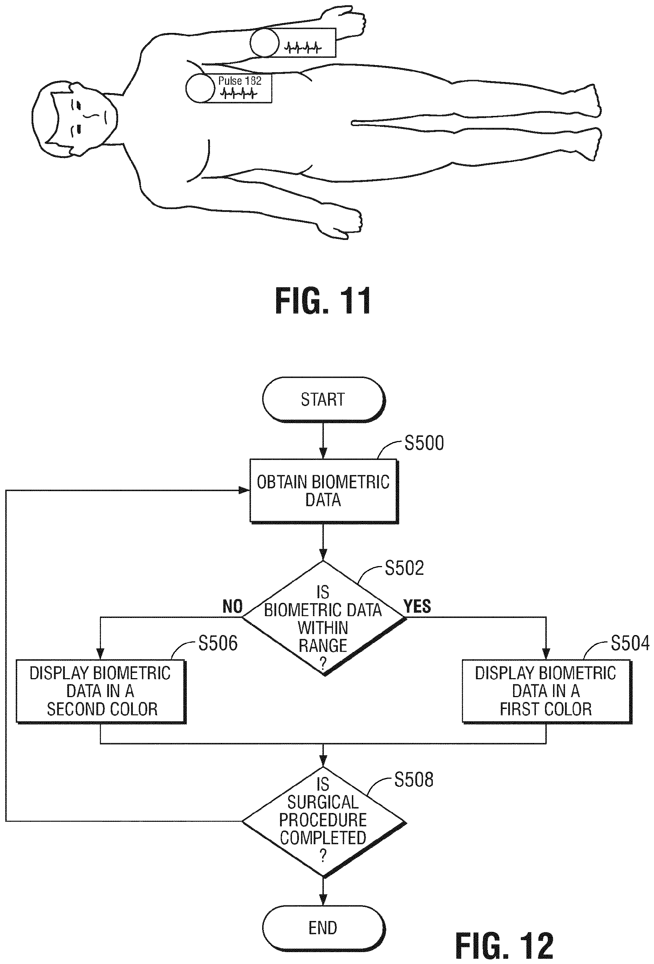

System 100 may also be used to display biometric data in the augmented image as shown in Fig. 11. For instance, the augmented image may include the patient's pulse and blood pressure that is obtained from sensor array 110. If the biometric data is within a normal range, e.g., the blood pressure as shown in Fig. 11, the biometric data may be a highlighted with a first color, e.g., green. If the biometric data is outside of a normal range, e.g., the pulse as shown in Fig. 11, the biometric data may be a highlighted with a second color, e.g., red.

Fig. 12 depicts a flowchart describing a process for displaying the biometric data. In step s500, the sensor array 110 obtains biometric data from the patient and provides the biometric data to controller 102. Controller 102 then determines whether the biometric data is within an acceptable range in step s502. If the biometric data is within an acceptable range, the process proceeds to step s504 where the biometric data is displayed in a first color, e.g., green. If the biometric data is not within an acceptable range, the process proceeds to step s506 where the biometric data is displayed in a second color, e.g., red. After steps s504 and s506, the controller determines whether the surgical procedure is completed in step s508. If the procedure is not completed, then the process returns to step s500. Otherwise, if the surgical procedure is completed, the process ends.

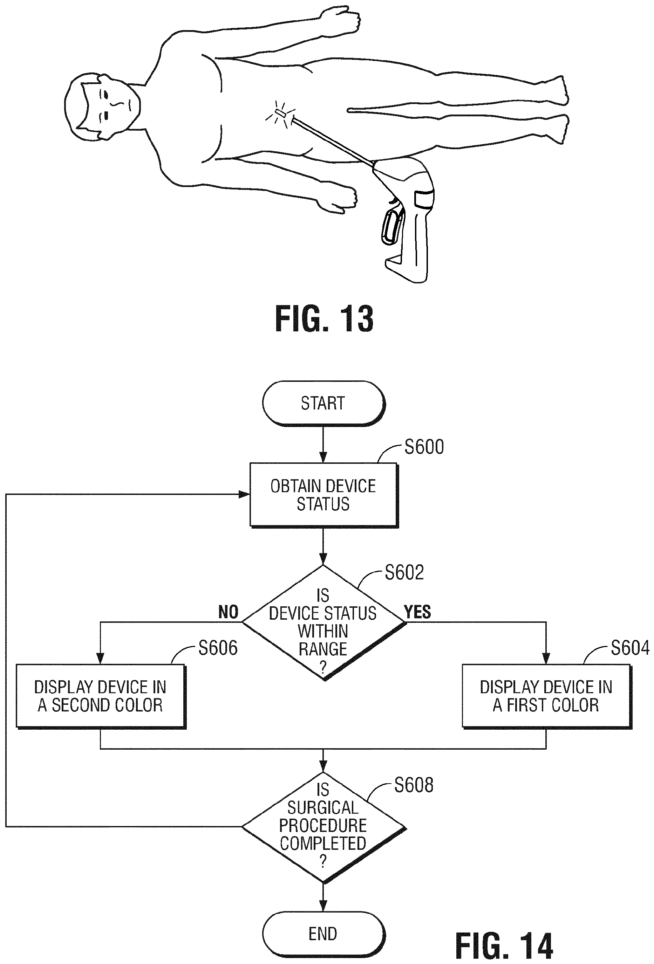

System 100 can also be used to display a surgical device status in the augmented image as shown in Fig. 13. For instance, the augmented image may highlight the device with a first color, e.g., green, if the status of the device is in an acceptable range. If the device is outside of a normal range, the device may be a highlighted with a second color, e.g., red. The status of the device may include, but is not limited to, firing range, remaining device life, battery charge, tissue thickness, tissue impedance, etc.

Fig. 14 depicts a flowchart describing a process for displaying the status. In step s600, the sensor array 110 obtains the status from the surgical device and provides the status to controller 102. Controller 102 then determines whether the status is within an acceptable range in step s602. If the status is within an acceptable range, the process proceeds to step s604 where the surgical device is displayed in a first color, e.g., green. If the status is not within an acceptable range, the process proceeds to step s606 where the surgical device is displayed in a second color, e.g., red. After steps s604 and s606, the controller determines whether the surgical procedure is completed in step s608. If the procedure is not completed, then the process returns to step s600. Otherwise, if the surgical procedure is completed, the process ends.

In other embodiments of the present disclosure, memory 106 may store a surgical plan to be used during a surgical procedure. The surgical plan may include a target area, a cut path, tools that are to be used during a surgical procedure and the order of use for such tools. Based on the surgical plan, the augmented image may provide the clinician with data to assist the clinician. For instance, in some embodiments, the clinician may make a first cut. Based on the magnitude and direction of the first cut, as well as data from the anatomical images, the controller may highlight a path on the augmented image for the clinician to make a second and subsequent cuts. In other embodiments, if the cut is quite large, the controller 102 will suggest a reload size or number of reloads that are necessary to perform the procedure.

Further, in some embodiments, the controller may determine which tool among the plurality of tools in the surgical plan has been used based on images from the image capture device 108. The controller 102 will then check the surgical plan to determine which tool will be used next by the clinician. The controller 102 then locates the tool in the image of the surgical environment and highlights the tool in the corresponding augmented image. Thus permitting scrub techs to be ready with the next tool when required by the clinician.

The controller 102 may also highlight areas in the augmented image that are in constant flux. The image capture device 108 captures a first image and a second image that is transmitted to controller 102. Controller 102 then determines whether a region in the second image has changed from the corresponding region in the first image. If the region has changed, the controller 102 highlights the corresponding region in the augmented image while dimming the other regions in the augmented image. Thus, the clinician may focus on the highlighted region.

It should be understood that the foregoing description is only illustrative of the present disclosure. Various alternatives and modifications can be devised by those skilled in the art without departing from the disclosure. For instance, any of the augmented images described herein can be combined into a single augmented image to be displayed to a clinician. Accordingly, the present disclosure is intended to embrace all such alternatives, modifications and variances. The embodiments described with reference to the attached drawing figs. are presented only to demonstrate certain examples of the disclosure. Other elements, steps, methods and techniques that are insubstantially different from those described above and/or in the appended claims are also intended to be within the scope of the disclosure.

## Claims

1. An augmented surgical reality environment system (100) comprising:
an image capture device (108) configured to capture an image of a surgical environment;
at least one biometric sensor configured to obtain biometric data from a patient;
a controller (102) including:
a memory (106) configured to store a plurality of anatomical images;
a processor (104) configured to: (i) receive at least one of the captured images of the surgical environment, the biometric data, or one or more anatomical images from the plurality of anatomical images; and (ii) generate an augmented image from at least one of the captured images of the surgical environment, the biometric data, or the one or more anatomical images; and
a display device (112) configured to display the augmented image; **characterized in that**:
the captured image of the surgical environment includes a direction and magnitude of a first cut and the processor determines a desired cut path and a distance for a second or subsequent cut based on: (i) the direction and magnitude of the first cut; and (ii) the plurality of anatomical images stored in the memory.

2. The augmented surgical reality environment system according to claim 1, wherein the display device (112) is a projector; preferably wherein the display device includes:
a frame;
at least one lens; and
a projector configured to project the augmented image onto the lens.

3. The augmented surgical reality environment system according to claim 1 or claim 2, wherein the controller (102) determines a position or orientation of a surgical tool relative to the patient; preferably wherein the augmented image includes a virtual image of a portion of the surgical tool disposed within the patient.

4. The augmented surgical reality environment system according to claim 3, wherein the position or orientation of the surgical tool is determined based on an image of the surgical tool captured by the image capture device (108); and/or wherein the position or orientation of the surgical tool is determined by data provided by the surgical tool; preferably wherein the data provided by the surgical tool includes accelerometer data or gyroscopic data.

5. The augmented surgical reality environment system according to any preceding claim, wherein the processor (104) determines a position of an object relative to the patient based on the image of the surgical environment; preferably wherein the augmented image includes an enhanced representation of the object and the display device (112) displays the enhanced representation of the object on the patient at the position determined by the processor.

6. The augmented surgical reality environment system according to any preceding claim, wherein the controller (102) is configured to receive a position signal from an object; preferably wherein the processor (104) determines a position of the object based on the received position signal.

7. The augmented surgical reality environment system according to claim 6, wherein the augmented image includes an enhanced representation of the object and the display device displays the enhanced representation of the object on the patient at the position determined by the processor.

8. The augmented surgical reality environment system according to any preceding claim, wherein the plurality of anatomical images are obtained from an x-ray, a computed tomography scan, or magnetic resonance imaging data; preferably wherein the anatomical images are processed by the processor (104) to enhance a portion of the anatomical image.

9. The augmented surgical reality environment system according to claim 8, wherein the enhanced portion of the anatomical image is displayed on the patient by the display device (112); preferably wherein the enhanced portion of the anatomical image includes a heat map.

10. The augmented surgical reality environment system according to any preceding claim, wherein the augmented image includes a surgical plan that includes one or more of a cut path, an incision location, an implant location, or notes.

11. The augmented surgical reality environment system according to any preceding claim, including a surgical device, wherein the augmented image includes a status of the surgical device.

12. The augmented surgical reality environment system according to claim 1, wherein the augmented image includes an image representing a direction and magnitude of the second cut.

13. The augmented surgical reality environment system according to any preceding claim, wherein the image capture device captures a first image of the surgical environment and a second image of the surgical environment,
wherein the controller determines if an object has moved based on a difference between the first image and the second image, and
wherein the controller highlights the object in the augmented image to be displayed on the display.

## Patentansprüche

1. System der erweiterten Realität einer chirurgischen Umgebung (100) umfassend:
eine Bilderfassungsvorrichtung (108) konfiguriert zum Erfassen eines Bilds einer chirurgischen Umgebung;
mindestens einen biometrischen Sensor konfiguriert zum Erhalt biometrischer Daten von einem Patienten;
einen Controller (102) umfassend:
einen Speicher (106) konfiguriert zum Speichern einer Vielzahl anatomischer Bilder;
einen Prozessor (104) konfiguriert um: (i) mindestens eines der erfassten Bilder der chirurgischen Umgebung, der biometrischen Daten oder eins oder mehr der anatomischen Bilder aus der Vielzahl anatomischer Bilder zu empfangen; und (ii) ein erweitertes Bild von wenigstens einem der erfassten Bilder der chirurgischen Umgebung, der biometrischen Daten oder dem einen oder mehreren anatomischen Bildern zu erzeugen; und
eine Anzeigevorrichtung (112) konfiguriert, um das erweiterte Bild anzuzeigen, **dadurch gekennzeichnet, dass**:
das erfasste Bild der chirurgischen Umgebung eine Richtung und einen Umfang eines ersten Schnitts umfasst und der Prozessor einen ersten Schnittpfad festlegt, sowie einen Abstand für einen zweiten oder nachfolgenden Schnitt basierend auf: (i) der Richtung und dem Umfang des ersten Schnitts; und (ii) der Vielzahl anatomischer Bilder, die in dem Speicher gespeichert sind.

2. System der erweiterten Realität einer chirurgischen Umgebung nach Anspruch 1, wobei die Anzeigevorrichtung (112) ein Projektor ist; vorzugsweise wobei die Anzeigevorrichtung umfasst:
einen Rahmen;
mindestens eine Linse; und
einen Projektor konfiguriert, um das erweiterte Bild auf die Linse zu projizieren.

3. System der erweiterten Realität einer chirurgischen Umgebung nach Anspruch 1 oder Anspruch 2, wobei der Controller (102) eine Position oder Ausrichtung eines chirurgischen Instrument in Bezug zu einem Patienten festlegt; vorzugsweise wobei das erweiterte Bild ein virtuelles Bild eines Teils des chirurgischen Instruments umfasst, das im Patienten angebracht ist.

4. System der erweiterten Realität einer chirurgischen Umgebung nach Anspruch 3, wobei die Position oder Ausrichtung des chirurgischen Instruments basierend auf einem Bild des chirurgischen Instruments festgelegt wird, das von der Bilderfassungsvorrichtung (108) erfasst wird; und/oder wobei die Position oder Ausrichtung des chirurgischen Instruments von Daten festgelegt wird, die das chirurgische Instrument liefert; vorzugsweise wobei die von dem chirurgischen Instrument gelieferten Daten Beschleunigungsmesserdaten oder gyroskopische Daten umfassen.

5. System der erweiterten Realität einer chirurgischen Umgebung nach einem der vorangegangenen Ansprüche, wobei der Prozessor (104) eine Position eines Objekts in Bezug zu einem Patienten basierend auf der chirurgischen Umgebung festlegt; vorzugsweise wobei das erweiterte Bild eine vergrößerte Darstellung des Gegenstands umfasst und die Anzeigevorrichtung (112) die vergrößerte Darstellung des Gegenstands an dem Patienten an der Position anzeigt, die von dem Prozessor festgelegt wurde.

6. System der erweiterten Realität einer chirurgischen Umgebung nach einem der vorangegangenen Ansprüche, wobei der Controller (102) konfiguriert ist, um ein Positionssignal von einem Gegenstand zu empfangen; vorzugsweise wobei der Prozessor (104) eine Position des Gegenstands basierend auf dem empfangenen Positionssignal festlegt.

7. System der erweiterten Realität einer chirurgischen Umgebung nach Anspruch 6, wobei das erweiterte Bild eine vergrößerte Darstellung des Gegenstands umfasst und die Anzeigevorrichtung die vergrößerte Darstellung des Gegenstands an dem Patienten an der von dem Prozessor festgelegten Position anzeigt.

8. System der erweiterten Realität einer chirurgischen Umgebung nach einem der vorangegangenen Ansprüche, wobei die Vielzahl an anatomischen Bildern aus Daten aus Röntgenaufnahmen, Computertomographie oder Magnetresonanz erhalten werden; vorzugsweise wobei die anatomischen Bilder durch den Prozessor (104) verarbeitet werden, um einen Teil des anatomischen Bilds zu vergrößern.

9. System der erweiterten Realität einer chirurgischen Umgebung nach Anspruch 8, wobei der vergrößerte Teil des anatomischen Bilds auf dem Patienten von der Anzeigevorrichtung (112) angezeigt wird; vorzugsweise wobei der vergrößerte Teil des anatomischen Bilds eine Heatmap umfassen.

10. System der erweiterten Realität einer chirurgischen Umgebung nach einem der vorangegangenen Ansprüche, wobei das erweiterte Bild einen chirurgischen Plan umfasst, der einen oder mehrere Schnittpfade, eine Einschnittstelle, eine Implantationsstelle oder Anmerkungen umfasst.

11. System der erweiterten Realität einer chirurgischen Umgebung nach einem der vorangegangenen Ansprüche, einschl. einer chirurgischen Vorrichtung, wobei das erweiterte Bild einen Status der chirurgischen Vorrichtung umfasst.

12. System der erweiterten Realität einer chirurgischen Umgebung nach Anspruch 1, wobei das erweiterte Bild ein Bild umfasst, das eine Richtung und einen Umfang des zweiten Schnitts umfasst.

13. System der erweiterten Realität einer chirurgischen Umgebung nach einem der vorangegangenen Ansprüche, wobei die Bilderfassungsvorrichtung ein erstes Bild der chirurgischen Umgebung erfasst und ein zweites Bild der chirurgischen Umgebung,
wobei der Controller festlegt, ob ein Gegenstand sich bewegt hat, basierend auf einer Differenz zwischen dem ersten Bild und dem zweiten Bild und
wobei der Controller den Gegenstand in dem erweiterten Bild hervorhebt, damit dieser auf der Anzeige angezeigt wird.

## Revendications

1. Système d'environnement de réalité chirurgicale augmentée (100) comprenant :
un dispositif de capture d'image (108) configuré pour capturer une image d'un environnement chirurgical ;
au moins un capteur biométrique configuré pour obtenir des données biométriques d'un patient ;
un dispositif de commande (102) comprenant :
une mémoire (106) configurée pour stocker une pluralité d'images anatomiques ;
un processeur (104) configuré pour : (i) recevoir au moins l'une des images capturées dans l'environnement chirurgical, des données biométriques, ou une ou plusieurs images anatomiques de la pluralité d'images anatomiques; et (ii) générer une image augmentée d'au moins l'une des images capturées de l'environnement chirurgical, des données biométriques, ou des une ou plusieurs images anatomiques ; et
un dispositif d'affichage (112) configuré pour afficher l'image augmentée ; **caractérisé en ce que** :
l'image capturée de l'environnement chirurgical comprend une direction et une magnitude d'une première coupe et le processeur détermine une trajectoire de coupe souhaitée et une distance pour une deuxième, ou suivante, coupe en se basant sur : (i) la direction et la magnitude de la première coupe; et (ii) la pluralité d'images anatomiques stockées dans la mémoire.

2. Système d'environnement de réalité chirurgicale augmentée selon la revendication 1, dans lequel le dispositif d'affichage (112) est un projecteur; de préférence dans lequel le dispositif d'affichage comprend :
un châssis ;
au moins un objectif ; et
un projecteur configuré pour projeter l'image augmentée sur l'objectif.

3. Système d'environnement de réalité chirurgicale augmentée selon la revendication 1 ou la revendication 2, dans lequel le dispositif de commande (102) détermine une position ou une orientation d'un outil chirurgical relativement au patient; de préférence dans lequel l'image augmentée comprend une image virtuelle d'une partie de l'outil chirurgical disposé à l'intérieur du patient.

4. Système d'environnement de réalité chirurgicale augmentée selon la revendication 3, dans lequel la position ou l'orientation de l'outil chirurgical est déterminée sur la base d'une image de l'outil chirurgical capturée par le dispositif de capture d'image (108) ; et/ou dans lequel la position ou l'orientation de l'outil chirurgical est déterminée par des données fournies par l'outil chirurgical ; de préférence dans lequel les données fournies par l'outil chirurgical comprennent des données d'accéléromètre ou des données gyroscopiques.

5. Système d'environnement de réalité chirurgicale augmentée selon l'une quelconque des revendications précédentes, dans lequel le processeur (104) détermine une position d'un objet relativement au patient sur la base de l'image de l'environnement chirurgical ; de préférence dans lequel l'image augmentée comprend une représentation de l'objet et le dispositif d'affichage (112) affiche la représentation améliorée de l'objet sur le patient au niveau de la position déterminée par le processeur.

6. Système d'environnement de réalité chirurgicale augmentée selon l'une quelconque des revendications précédentes, dans lequel le dispositif de commande (102) est configuré pour recevoir un signal de position d'un objet ; de préférence dans lequel le processeur (104) détermine une position de l'objet sur la base du signal de position reçu.

7. Système d'environnement de réalité chirurgicale augmentée selon la revendication 6, dans lequel l'image augmentée comprend une représentation améliorée de l'objet et le dispositif d'affichage affiche la représentation améliorée de l'objet sur le patient au niveau de la position déterminée par le processeur.

8. Système d'environnement de réalité chirurgicale augmentée selon l'une quelconque des revendications précédentes, dans lequel la pluralité d'images anatomiques sont obtenues d'un rayon X, d'un tomodensitomètre, ou de données d'imagerie de résonance magnétique; de préférence dans lequel les images anatomiques sont traitées par le processeur (104) pour améliorer une partie de l'image anatomique.

9. Système d'environnement de réalité chirurgicale augmentée selon la revendication 8, dans lequel la partie améliorée de l'image anatomique est affichée sur le patient par le dispositif d'affichage (112) ; de préférence dans lequel la partie améliorée de l'image anatomique comprend une carte thermique.

10. Système d'environnement de réalité chirurgicale augmentée selon l'une quelconque des revendications précédentes, dans lequel l'image augmentée comprend un plan chirurgical qui comprend une ou plus d'une trajectoire de coupe, d'un emplacement d'incision, d'un emplacement d'implant, ou de notes.

11. Système d'environnement de réalité chirurgicale augmentée selon l'une quelconque des revendications précédentes, comprenant un dispositif chirurgical, dans lequel l'image augmentée comprend un statut du dispositif chirurgical.

12. Système d'environnement de réalité chirurgicale augmentée selon la revendication 1, dans lequel l'image augmentée comprend une image représentant une direction et une magnitude de la seconde coupe.

13. Système d'environnement de réalité chirurgicale augmentée selon l'une quelconque des revendications précédentes, dans lequel le dispositif de capture d'image capture une première image de l'environnement chirurgical et une seconde image de l'environnement chirurgical,
dans lequel le dispositif de commande détermine si un objet s'est déplacé sur la base d'une différence entre la première image et la deuxième image, et
dans lequel le dispositif de commande met en évidence l'objet sur l'image augmentée à afficher sur l'écran.
